# FASCICULE DE BREVET EUROPEEN

(11) **EP 1 795 181 B1**
(45) Date de publication et mention de la délivrance du brevet: **24.07.2013**
(21) Numéro de dépôt: 06125317.5
(22) Date de dépôt: 04.12.2006
(51) Int. Cl.: A61K 8/64, A61K 8/67, A61K 31/455, A61K 36/23, A61K 36/738, A61K 38/05, A61P 17/00, A61P 17/04, A61P 17/06, A61P 17/14, A61P 31/22, A61Q 7/00, A61Q 19/00, A61Q 19/02

(54) **Utilisations cosmétiques et pharmaceutiques de l'association du dipeptide tyrosine-arginine et de la niacinamide en tant qu'antagoniste de substance P**
Kosmetische und pharmazeutische Verwendungen des Dipeptids Tyrosin-Arginin in Kombination mit Niacinamid als Substanz-P-Antagonisten
Cosmetic and pharmaceutical uses of the dipeptide tyrosine-arginine in combination with niacinamide as substance P antagonist

(30) Priorité: 05.12.2005 FR 0553729
(43) Date de publication de la demande: 13.06.2007
(73) Titulaire: L'Oréal, 75008 Paris (FR)
(72) Inventeur: Potin, Anthony, 75006, Paris (FR)
(74) Mandataire: Brohmi, Karim

(56) Documents cités:
- EP-A- 0 583 479
- WO-A-00/02593
- WO-A-94/09750
- WO-A-98/07744
- WO-A-98/09985
- WO-A-2004/000333
- DE-C1- 4 018 964
- US-A1- 2005 053 572
- US-B1- 6 444 647
- LEE W.S.; SOHN I.B.: "Substance P prolongs human hair growth in vitro", JOURNAL OF DERMATOLOGICAL SCIENCE, vol. 33, 2003, pages 137-138,
- AHN H.J. ET AL: "Combined effect of substance P and calcitonin gene related peptide on the hair growth", JOURNAL OF INVESTIGATIVE DERMATOLOGY SYMPOSIUM PROCEEDINGS, vol. 8, no. 1, June 2003 (2003-06), page 139,
- DATABASE EMBASE ELSEVIER SCIENCE PUBLISHERS, AMSTERDAM, NL Décembre 2004 OH T.H. ET AL: 'Effects of substance P on the expression of various factors to control hair growth in human hair follicle culture'

## Description

La présente invention a pour domaine technique le traitement cosmétique ou dermatologique des peaux et/ou des muqueuses et/ou des cuirs chevelus.

L'invention a pour objet l'utilisation de l'association synergique du dipeptide tyrosine-arginine tel que défini dans les revendications et de la niacinamide en tant qu'antagoniste de substance P, pour la préparation d'une composition pour traiter les désordres associés à un excès de synthèse et/ou de libération de substance P définis dans les revendications.
L'invention a également pour objet l'utilisation de l'association du dipeptide tyrosine-arginine tel que défini dans les revendications et de la niacinamide pour traiter les peaux sensibles ainsi qu'un procédé cosmétique de traitement des peaux sensibles.

Il existe chez les mammifères des polypeptides appartenant à la famille des tachykinines qui induisent sur les fibres musculaires lisses des contractions rapides. Parmi les composés de cette famille, on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de nombreux désordres sont associés à un excès de synthèse et/ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonies) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple la rosacée, le psoriasis, les maladies prurigineuses, l'herpès, les photodermatoses, les dermatites atopiques, les dermatites de contact, les lichens, les prurits, les prurigos, les érythèmes en particulier solaire, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales, dans certaines maladies dermatologiques (telles que l'eczéma) et dans les affections ophtalmologiques (telles que par exemple les conjonctivites, les uvéites, les prurits oculaires, les douleurs oculaires, les irritations).

Plus spécifiquement, lorsqu'elle est libérée au niveau de la peau, la substance P exerce une vasodilatation et une extravasation plasmatique pouvant induire rougeur cutanée et oedème.

L'utilisation d'antagoniste de substance P est un moyen efficace de prévenir et/ou atténuer et/ou traiter les manifestations citées ci-dessus.

Par antagoniste de substance P, on entend tout composé susceptible d'inhiber partiellement, voire totalement, l'effet biologique de la substance P.
Particulièrement, pour qu'une substance soit reconnue comme un antagoniste de substance P elle doit induire une réponse pharmacologique cohérente (incluant ou non sa fixation au récepteur de la substance P) par exemple dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induite par l'administration de substance P.

Il a également été mis en évidence que la substance P pouvait être responsable de manifestations cutanées caractérisant les peaux sensibles, par peau, on entend toute surface cutanée du corps incluant le cuir chevelu ainsi que les muqueuses et semi-muqueuses (telles que les lèvres).

D'une manière générale, les peaux sensibles se définissent par une réactivité particulière de la peau.
Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultraviolets ou infrarouges. Il existe également des facteurs associés comme l'âge et le type de peau. Ainsi les peaux sensibles sont plus fréquentes parmi les peaux sèches ou grasses que parmi les peaux normales.
L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations plus ou moins douloureuses ressenties dans une zone cutanée comme les picotements, fourmillements, démangeaisons, brûlures, échauffements, inconforts, tiraillements, etc. Ces signes subjectifs existent le plus souvent en l'absence de signes cliniques visibles tels que la rougeur et les desquamations et en l'absence de réaction inflammatoire. On sait aujourd'hui que ces réactions d'irritation et d'intolérance cutanée sont notamment liées à une libération de neuropeptides par les terminaisons nerveuses de l'épiderme et du derme.

Par opposition aux peaux qualifiées d'allergiques, la réactivité d'une peau sensible ne relève pas d'un processus immunologique. Son mécanisme de réponse est dit "aspécifique". Elle est, à ce titre, à distinguer des peaux manifestant des réactions inflammatoires et allergiques de type dermatose, eczéma, et/ou ichtyose, et vis-à-vis desquelles un certain nombre de traitements ont déjà été proposés.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible, de l'irritation ou de l'allergie de contact pour lesquelles il existe en revanche des signes inflammatoires visibles.

En conséquence, la mise au point de produits "peaux sensibles" a nécessité de disposer d'outils d'évaluation de la réaction sensorielle de la peau. Les premiers outils se sont inspirés dès leur conception de la caractéristique essentielle des peaux sensibles à savoir la présence de signes d'inconfort induits par une application topique.
Ainsi, le "stinging test" à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées "stingers" et considérés comme étant à peau sensible. En raison de cette sensibilité cutanée à l'application topique de produit, ces sujets sont alors sélectionnés pour tester des produits dits peaux sensibles.
Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.
Ce second type de test, décrit dans la demande EP 1 374 913, constitue également un autre moyen particulièrement utile pour le diagnostic de peaux sensibles.

Au sens de la présente invention, les peaux sensibles couvrent les peaux irritables et les peaux intolérantes.
Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.
Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Les cuirs chevelus "sensibles" ont une sémiologie clinique plus univoque : les sensations de démangeaisons et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

La demanderesse a mis en évidence que l'association du dipeptide tyrosine-arginine et de la niacinamide présente une activité significativement améliorée en tant qu'antagoniste de substance P, par rapport à l'activité de chacun de ces actifs pris séparément. Il est donc apparu comme avantageux d'utiliser l'association niacinamide et dipeptide pour le traitement des désordres liés à une libération excessive de substance P.

L'invention a ainsi pour objet l'utilisation, dans une composition cosmétique ou pharmaceutique, en quantité efficace du dipeptide tyrosine-arginine et de niacinamide en tant qu'antagoniste de substance P.

Le dipeptide tyrosine-arginine est connu pour ses propriétés antalgiques et apaisantes (WO 98/07744), il a également été décrit comme myorelaxant.

WO 98/09985 décrit l'utilisation de différents dipeptides à titre d'agent inhibiteur de l'activité des lymphocytes et des macrophages et comme anti-inflammatoire en vue, notamment, du traitement ou de l'amélioration de maladies du collagène ou des dermes.

Le dipeptide correspond à la formule générale suivante : R1-L-Tyr-L-Arg-R2 avec
- R1 est un atome d'hydrogène ou un groupe -R3-C=O et R3 est une chaîne alkyle de 1 à 20 atomes de carbones, linéaire ou ramifiée, saturée ou insaturée, éventuellement hydroxylée, ou un groupe aryle, un groupe aryle-alkyle, un groupe alkyloxy ou un groupe arylalkyloxy et
- R2 est une fonction hydroxyle ; un groupe -O-R4 avec R4 est une chaîne alkyle de 1 à 20 atomes de carbones ; un groupe -NH2 ou -NHX avec X est une chaîne alkyle de 1 à 4 atomes de carbones.

Il se peut que pour des questions de résistance à la dégradation, il soit avantageux d'utiliser une forme protégée du peptide. La forme de la protection doit être une forme biologiquement compatible. De nombreuses formes de protection biologiquement compatibles peuvent être envisagées comme par exemple l'acylation ou l'acétylation de l'extrémité amino-terminale ou l'amidation de l'extrémité carboxy-terminale.

De préférence, on utilisera le N-acétyl-Tyrosine-Arginine-O-hexadecyl ester qui présente des propriétés de lipophilie, de stabilité et de bio-disponibilité avantageuses.

La synthèse du dipeptide et de ses formes modifiées est décrite dans le WO 98/07744.

On désignera par la suite indifféremment par « dipeptide » ou « dipeptide tyrosine-arginine » ou « dipeptide tyr-arg », le dipeptide tyrosine-arginine tel que défini dans les revendications.

La niacinamide aussi appelée acide nicotinique, nicotinamide ou encore vitamine B3, est la pyridinecarboxamide-3, membre de la famille des 8 vitamines B hydrosolubles.
La vitamine B3 est nécessaire à la respiration cellulaire, aide au métabolisme énergétique et des glucides, lipides et protéines, à la circulation sanguine. Cette vitamine est également connue pour présenter des propriétés anti-inflammatoires.
WO 2004/000333 propose la mise en oeuvre d'une combinaison d'un ester d'acide gras d'un polyhydroxyalcane et d'un dérivé d'une carboxypyridine à des fins de traitements des troubles cutanés.
Les symptômes d'une carence en vitamine B3 comprennent indigestion, fatigue, voire même vomissements et dépression.

Plus particulièrement, l'association niacinamide et dipeptide tyrosine-arginine peut être utilisée pour prévenir et/ou atténuer l'intensité des rougeurs cutanées et/ou les oedèmes cutanés légers, éclaircir et/ou uniformiser le teint et/ou masquer les rougeurs de surface et/ou estomper les signes de la microcirculation cutanée, c'est-à-dire rendre moins visibles les microvaisseaux sanguins apparents notamment sur le visage.

L'association niacinamide et dipeptide pourra encore être utilisée pour éviter un aspect gonflé de la peau provoqué par des oedèmes légers et, par exemple, être formulée dans des soins pour affiner la silhouette, le cou et/ou l'ovale du visage, diminuer les poches sous les yeux, traiter les chevilles et les jambes gonflées...

L'utilisation de l'association niacinamide et dipeptide tyrosine-arginine pourra aussi être destinée au traitement des peaux sensibles, notamment, les peaux intolérantes ou irritables, pour prévenir et/ou diminuer les sensations dysesthésiques, pour prévenir et/ou diminuer les picotements et/ou les fourmillements et/ou les démangeaisons et/ou les échauffements et/ou l'inconfort cutané et/ou les tiraillements de la peau.

Un autre objet de la présente invention se rapporte à l'utilisation d'une quantité efficace de niacinamide et du dipeptide tyrosine-arginine pour la préparation d'une composition destinée à traiter les désordres associés à un excès de synthèse et/ou de libération de substance P, lesdits désordres étant des désordres cutanés choisis parmi l'urticaire, les dermatites eczémateuses, la rosacée, le psoriasis, l'herpes, les photodermatoses, la dermatite atopique, la dermatite de contact, le lichen, les prurigos, les maladies prurigineuses, les fibroses, les troubles de la maturation du collagène, la sclérodermie, l'eczéma.

L'association de niacinamide et de dipeptide tyrosine-arginine est particulièrement adaptée au traitement des signes de la rosacée, notamment de son premier stade.
La rosacée est une dermatose commune chronique et progressive liée à une relaxation vasculaire. Elle affecte principalement la partie centrale du visage et se caractérise par le rougissement du visage ou les bouffées de chaleur, l'érythème facial, les papules, les pustules, et la télangiectasie. Dans les cas graves, particulièrement chez l'homme, le tissu mou du nez peut enfler et produire un gonflement bulbeux appelé rhinophyma.
La rosacée évolue généralement en 4 stades :
- stade 1 des relaxations vasculaires ayant une composante neurogène (vers 20 ans). Les patients ont des poussées soudaines de rougeur paroxystique du visage et du cou, avec sensation de chaleur, mais sans signes systémiques. Après les crises, la peau du visage redevient normale. Ces « flushes » sont déclenchés par les changements de température (entraînant parfois une thermophobie), l'absorption de boissons chaudes ou d'alcool.
- stade 2 érythémato-télangiectasique (vers 30 ans). Les zones malaires sont diffusément rouges. On y observe des capillaires dilatés constituant la classique couperose. A la différence du stade 1, la rougeur est permanente. Outre les joues, le menton et la partie médiane du front peuvent être touchés.
- stade 3 des papulo-pustules (vers 40 ans). Sur un fond d'érythème se développent des papules et des pustules de quelques millimètres de diamètre, sans comédons associés. Cette dermatose peut être très étendue, parfois à la partie glabre du cuir chevelu chez l'homme, mais respecte le pourtour de la bouche et des yeux. Les patients se plaignent d'une peau sensible, avec intolérance subjective à la plupart des topiques et des cosmétiques gras.
- stade 4 du rhinophyma (vers 50 ans ou plus tard). Cette phase tardive touche principalement les hommes, contrairement aux autres stades. Le nez est augmenté de volume, diffusément rouge et les orifices folliculaires sont dilatés. La peau s'épaissit progressivement.

La présente invention a également pour objet un procédé de traitement cosmétique, caractérisé en ce que l'on applique une quantité efficace de l'association dipeptide tyrosine-arginine et niacinamide sur la peau, sur le cuir chevelu et/ou sur les muqueuses des zones cutanées pour prévenir et/ou estomper les rougeurs et/ou les signes de la microcirculation cutanée et/ou les oedèmes cutanés.

La présente invention se rapporte également à un procédé cosmétique de traitement des peaux sensibles caractérisé en ce que l'on applique une quantité efficace de l'association niacinamide et dipeptide tyrosine-arginine sur la peau, sur le cuir chevelu et/ou sur les muqueuses.

On a vu précédemment ce qu'est une peau irritable. L'irritation cutanée peut avoir de multiples causes. Ce peut être des causes intrinsèques, liées au dérèglement des mécanismes physiologiques conduisant à une peau normale. Mais ce peut être également des causes extrinsèques comme des composés irritants qui viendraient en contact avec la peau.

Ainsi, la présente invention a encore pour objet un procédé cosmétique en vue de diminuer l'irritation cutanée, caractérisé par le fait que l'on utilise par application sur la peau, sur les cheveux, et/ou sur les muqueuses, l'association de niacinamide et de dipeptide.

Les procédés cosmétiques de l'invention peuvent être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de sticks, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les quantités efficaces de niacinamide et de dipeptide correspondent à la quantité nécessaire de chacun des deux composés pour présenter un effet amélioré. Les tests mis en oeuvre dans le cadre de la présente invention (voir les exemples 1 et 2) ont permis de mettre en évidence de façon surprenante que l'association niacinamide et dipeptide conduit à une activité antagoniste de substance P bien supérieure que l'activité de ces actifs pris séparément.
Plus particulièrement, dans les compositions selon l'invention comprenant dans un milieu physiologiquement acceptable, le dipeptide est présent dans des concentrations comprises entre 0,001 % à 20%, de préférence, entre 0,01 % à 10 % et la niacinamide est présente dans des concentrations comprises entre 0,01 % à 20 %, de préférence, entre 0,1 % à 10 %.

De façon avantageuse, la composition selon l'invention comprendra en outre au moins un agent apaisant.
Comme exemples 'd'agents apaisants' utilisables dans les compositions de l'invention, on peut citer:
- l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ;
- les planctons, lyophilisés ou non, leurs extraits et leurs complexes ;
- l'escine et les extraits végétaux en contenant comme l'extrait de marron d'inde ;
- les dérivés de xanthine comme le chlorhydrate de di-éthylaminoéthyl théophylline et la caféine ;
- les eaux et extraits (par exemple aqueux, hydroalcooliques ou hydroglycoliques) de fleurs et de plantes, comme l'eau de bleuet, l'eau de camomille, l'eau de menthe, l'eau de tilleul, l'eau de rose, les extraits de Rosacées (ex : *Rosa gallica*), les extraits de pivoine, les extraits d'aubépine, les extraits de millefeuille, les extraits de mauve, les extraits de souci, les extraits de melilot, les extraits de sauge, l'eau de sureau, les extraits de ginkgo biloba, les extraits d'arnica, les extraits d'origan, les extraits de thé vert, les extraits de fleurs de nénuphar, les extraits d'Iris, les extraits d'écorce de bouleau, les extraits d'Aloe vera ;
- l'acide asiatique et les extraits végétaux en contenant comme la Centella Asiatica ;
- les extraits de fruits, comme l'extrait d'ananas, l'extrait de papaye ; de goyave ;
- les algues notamment du type *Laminaria* (par exemple rouges ou brunes) ;
- les pyrrolidone carboxylates et notamment de zinc (Zn-PCA) ou de cuivre (Cu-PCA) ;
- les huiles d'origine végétale, comme l'huile de graine de canola et l'huile de karité ;
- les huiles essentielles, par exemple de coriandre, de mélisse, de lavande, de menthe, de camomille et leurs mélanges ;
- l'acide acexamique et l'acide transexamique (acide trans-4, aminométhylcyclohexane carboxylique) ;
- l'acide ursolique et les extraits en contenant comme l'extrait de feuille de romarin ;
- les polysaccharides contenant du fucose, comme le FUCOGEL 1000, vendu par Solabia (solution aqueuse comprenant 1% de matière sèche de polysaccharide comprenant du fucose, du galactose et de l'acide galacturonique) ;
- les électrolytes et en particulier un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange étant ici appelé "mélange des sels de la mer Morte" (« Dead Sea Bath Salts ») car il correspond aux principaux sels contenus dans la mer Morte ;
- les galactolipides par exemple issus d'avoine, tels que par exemple le digalactosyl diglycéride ou le monogalactosyl diglycéride ;
- les acides aminés, leurs dérivés et leurs sels, tels que le sel de sodium d'aminoacides greffés sur des chaînes cocoyle, commercialisé sous forme d'un mélange sous la dénomination SEPICALM S par la société SEPPIC, le capryloylglycine commercialisé sous la dénomination LIPACIDE C8G par la société SEPPIC et le mélange de capryloylglycine, de cannelle et de sarcosine commercialisé sous la dénomination SEPICONTROL A5 par la société SEPPIC ;
- les antagonistes de TNF-alpha tels que la lisophylline, l'A802715, la sulfasalazine, le CDP-571 (anticorps anti-TNF-alpha), le MDL-201112 ;
- les antagonistes de substance P tels que le sendide, le spantide II, et les peptides décrits dans la demande EP-A-680749, les extraits de bactérie filamenteuse décrits dans la demande EP-A-761204 ;
- les antagonistes de CGRP, tels que le CGRP 8-37, les anticorps anti-CGRP, ou des extraits végétaux à activité antagoniste du CGRP (ex *: Iris pallida*).
- les sels divalents de strontium, de zinc, de manganèse, de magnésium, de calcium, tels que ceux décrits dans les documents WO-A-96/19184, WO-A-96/19182 et WO-A-96/19228 ;
   et leurs mélanges.

De préférence, l'agent apaisant pourra être choisi parmi les extraits de rose, le bisabolol, le D-panthenol, l'allantoïne, le madecassoside, les extraits de centella asiatica, le glycyrrhizinate de potassium ou la caféine.

La quantité d'agent(s) apaisant(s) peut aller par exemple de 0,001 à 20 % en poids et de préférence de 0,01 à 15 % en poids par rapport au poids total de la composition.

Selon la destination de la composition selon l'invention, elle pourra également comprendre des actifs qui seront choisis par l'homme du métier de telle sorte qu'ils ne nuisent pas à l'effet de l'association niacinamide et dipeptide.

Parmi ces actifs, on peut citer :
- les agents desquamants et hydratants ;
Par « agent desquamant », on entend tout composé capable d'agir :
- soit directement sur la desquamation en favorisant l'exfoliation, tel que les β -hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M ; le miel ; les dérivés de sucre tels que l'O-octanoyl-6-D-maltose et la N-acétyl glucosamine.
Par « agent hydratant », on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.
   Ces composés peuvent représenter de 0,001% à 30%, et de préférence de 0,01 à 20%, du poids total de la composition selon l'invention.
   La composition selon la présente invention comprenant les agents desquamants et hydratants cités ci-dessus est avantageusement destinée à la prévention ou au traitement du dessèchement de la peau et notamment des xéroses.
- les agents dépigmentants, anti-pigmentants ou pro-pigmentants ;
   Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.
   Comme agent pro-pigmentant, on peut citer l'extrait de pimprenelle (Sanguisorba officinalis) commercialisé par MARUZEN et les extraits de chrysanthème (Chrysanthemum morifolium).
- les agents anti-glycation ;
   Par « agent anti-glycation », on entend un composé prévenant et/ou diminuant la glycation des protéines de la peau, en particulier des protéines du derme telles que le collagène.
   Des exemples d'agents anti-glycation sont les extraits végétaux de la famille des Ericaceae, tels qu'un extrait de myrtille *(Vaccinium angusfifollium*) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 2 802 425, FR 2 810 548, FR 2 796 278 et FR 2 802 420, respectivement. Le resvératrol est particulièrement préféré pour une utilisation dans cette invention.
- les inhibiteurs de NO-synthase
   Des exemples d'inhibiteurs de NO-synthase convenant à une utilisation dans la présente invention comprennent notamment un extrait de végétal de l'espèce *Vitis vinifera* qui est notamment commercialisé par Euromed sous la dénomination Leucocyanidines de raisins extra, ou encore par Indena sous la dénomination Leucoselect^{®}, ou enfin par Hansen sous la dénomination Extrait de marc de raisin ; un extrait de végétal de l'espèce *Olea europaea* qui est de préférence obtenu à partir de feuilles d'olivier et est notamment commercialisé par VINYALS sous forme d'extrait sec, ou par Biologia & Technologia sous la dénomination commerciale Eurol BT ; et un extrait d'un végétal de l'espèce *Gingko biloba* qui est de préférence un extrait aqueux sec de ce végétal vendu par Beaufour sous le nom commercial Ginkgo biloba extrait standard.
   La composition selon l'invention comprenant un inhibiteur de NO-synthase tel que défini ci-dessus peut avantageusement être utilisée pour prévenir ou traiter les peaux sensibles.
- les agents anti-séborrhéiques ;
   Lorsque la composition selon l'invention comprend un agent anti-séborrhéique tel qu'un inhibiteur de 5α-réductase, celui-ci peut notamment être choisi parmi :
   - les rétinoïdes, et en particulier le rétinol ;
   - le soufre et les dérivés soufrés ;
   - les sels de zinc tels que le lactate, le gluconate, le pidolate, le carboxylate, le salicylate et/ou le cystéate de zinc ;
   - le chlorure de sélénium ;
   - la vitamine B6 ou pyridoxine ;
   - le mélange de capryloyl glycine, de sarcosine et d'extrait de cinnamomum zeylanicum commercialisé notamment par SEPPIC sous la dénomination commerciale Sepicontrol A5^{®} ;
   - un extrait de Laminaria saccharina commercialisé notamment par SECMA sous la dénomination commerciale Phlorogine^{®} ;
   - un extrait de Spiraea ulmaria commercialisé notamment par SILAB sous la dénomination commerciale Sebonormine^{®} ;
   - des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par MARUZEN ;
   - un extrait de Serenoa repens commercialisé notamment par EUROMED ;
   - des extraits de plantes du genre Silybum ; et
   - des extraits d'Eugenia caryophyllata contenant de l'eugénol et du glucoside d'eugényle.
      L'inhibiteur de 5α-réductase représente par exemple de 0,001 % à 10%, et de préférence de 0,01 à 5%, du poids total de la composition selon l'invention.
- les inhibiteurs de lysyl et/ou prolyl hydroxylase ;
   Des exemples préférés d'inhibiteurs de lysyl et/ou propyl hydroxylase utilisables dans la composition selon la présente invention sont le 2,4-diamino-pyrimidine 3-oxyde ou 2,4-DPO décrit dans la demande de brevet WO 96/09048 et le 2,4-diamino-6-pipéridino pyrimidine 3-oxyde ou "Minoxidil" décrit dans les brevets US-4,139,619 et US-4,596,812. Ces composés sont par exemple présents dans la composition selon l'invention à hauteur de 0,001 à 5% en poids et, mieux, à hauteur de 0,01 à 5% en poids, par rapport au poids total de la composition.
- les agents stimulant la synthèse de macromolécules dermiques ou épidermiques et/ou empêchant leur dégradation ;
   Parmi les actifs stimulant les macromolécules du derme ou empêchant leur dégradation, on peut citer ceux qui agissent :
   - soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par SEDERMA ; les peptides extraits de végétaux, tels que l'hydrolysat de soja commercialisé par COLETICA sous la dénomination commerciale Phytokine^{®} ; et les hormones végétales telles que les auxines et les lignanes.
   - soit sur la synthèse d'élastine, tels que l'extrait de *Saccharomyces cerivisiae* commercialisé par LSN sous la dénomination commerciale Cytovitin^{®} ; et l'extrait d'algue Macrocystis pyrifera commercialisé par SECMA sous la dénomination commerciale Kelpadelie^{®} ;
   - soit sur la synthèse des glycosaminoglycanes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par ALBAN MÜLLER sous la dénomination commerciale HSP3^{®} ; et l'extrait de *Saccharomyces cerevisiae* disponible notamment auprès de SILAB sous la dénomination commerciale Firmalift^{®} ou auprès de LSN sous la dénomination commerciale Cytovitin^{®} ;
   - soit sur la synthèse de la fibronectine, tels que l'extrait de zooplancton Salina commercialisé par SEPORGA sous la dénomination commerciale GP4G^{®} ;
      l'extrait de levure disponible notamment auprès de ALBAN MÜLLER sous la dénomination commerciale Drieline^{®} ; et le palmitoyl pentapeptide commercialisé par SEDERMA sous la dénomination commerciale Matrixil^{®} ;
   - soit sur l'inhibition des métalloprotéinases (MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par ICHIMARU PHARCOS sous la dénomination commerciale Flavostérone SB^{®}), de trèfle rouge, de lin, de kakkon ou de sauge ;
   - soit sur l'inhibition des sérine protéases telles que l'élastase leucocytaire ou la cathepsine G. On peut citer : l'extrait peptidique de graines de légumineuse (Pisum sativum) commercialisé par LSN sous la dénomination commerciale Parelastyl^{®} ; les héparinoïdes ; et les pseudodipeptides tels que l'acide {2-[acétyl-(3-trifluorométhyl-phényl)-amino]-3-méthyl-butyrylamino} acétique.
      Parmi les actifs stimulant les macromolécules épidermiques, telles que la fillagrine et les kératines, on peut citer notamment l'extrait de lupin commercialisé par SILAB sous la dénomination commerciale Structurine^{®} ; l'extrait de bourgeons de hêtre Fagus sylvatica commercialisé par GATTEFOSSE sous la dénomination commerciale Gatuline^{®} ; et l'extrait de zooplancton Salina commercialisé par SEPORGA sous la dénomination commerciale GP4G^{®}.
- les agents stimulant la prolifération des fibroblastes ou des kératinocytes et/ou la différenciation des kératinocytes ;
   Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par LSN sous la dénomination Eleseryl SH-VEG 8^{®} ou commercialisé par SILAB sous la dénomination commerciale Raffermine^{®}) ; et les hormones végétales telles que les giberrellines et les cytokinines.
   Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par GATTEFOSSE ; et les extraits de Solanum tuberosum commercialisés par SEDERMA.
   Les agents stimulant la différenciation des kératinocytes comprennent par exemple les minéraux tels que le calcium ; un extrait peptidique de lupin tel que celui commercialisé par SILAB sous la dénomination commerciale Structurine^{®}; le beta-sitosteryl sulfate de sodium tel que celui commercialisé par SEPORGA sous la dénomination commerciale Phytocohésine^{®} ; et un extrait hydrosoluble de maïs tel que celui commercialisé par SOLABIA sous la dénomination commerciale Phytovityl^{®} ; un extrait peptidique de *Voandzeia substerranea* tel que celui commercialisé par les Laboratoires Sérobiologiques sous la dénomination commerciale Filladyn LS 9397^{®} ; et les lignanes tels que le sécoisolaricirésinol.
- les agents myorelaxants ou dermo-décontractants ;
   Les agents myorelaxants ou dermo-décontractants utilisables dans la composition selon l'invention comprennent l'alvérine et ses sels, le gluconate de manganèse, le Diazepam, l'hexapeptide Argireline R commercialisé par LIPOTEC, certaines amines secondaires et tertiaires carbonylées, l'adénosine, ainsi que les sapogénines et les extraits naturels, en particulier de Wild Yam, en contenant, ainsi que les extraits de Boswellia serrata.
- les agents anti-microbiens :
   Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.
   Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.
   A titre d'exemple, l'agent antimicrobien peut être utilisé dans la composition selon l'invention en une quantité représentant de 0,1 à 20%, et de préférence de 0,1 à 10%, du poids total de la composition.
- les agents tenseurs ;
   Par "agent tenseur", on entend un composé capable d'exercer une traction sur la peau, qui a pour effet d'estomper temporairement les irrégularités de la surface de la peau, telles que les rides et ridules.
   Parmi les agents tenseurs utilisables dans la composition selon la présente invention, on peut citer notamment :
   (1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par 3M sous les dénominations commerciales VS 80, VS 70 ou LO21,
   (2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
   (3) les protéines et hydrolysats de protéines végétales, en particulier de maïs, de seigle, de froment, de sarrasin, de sésame, d'épautre, de pois, de fève, de lentille, de soja et de lupin,
   (3) les silicates mixtes, notamment les phyllosilicates et en particulier les Laponites,
   (4) les microparticules de cire, choisies par exemple parmi les cires de Carnauba, de Candelila ou d'Alfa,
   (5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.
- les agents anti-pollution ou anti-radicalaire :
   Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.
   Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par SOLABIA sous la dénomination commerciale Phytovityl^{®}, le mélange d'extrait de fumeterre et d'extrait de citron commercialisé sous la dénomination Unicotrozon C-49^{®} par Induchem, et le mélange d'extraits de ginseng, de pomme, de pêche, de blé et d'orge vendu par PROVITAL sous la dénomination commerciale Pronalen Bioprotect^{®}.
   Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par SOLABIA sous la dénomination commerciale Phytovityl^{®}.
   Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par SOLABIA sous la dénomination commerciale Phytovityl^{®}.
   Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le coenzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.
- les actifs lipolytiques ou ayant une activité favorable, directe ou indirecte, sur la diminution du tissu adipeux ;
   Parmi les dérivés susceptibles de favoriser la lipolyse on peut trouver :
   1) les inhibiteurs de phosphodiestérase, tels que :
      - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ;
      - les associations contenant des dérivés xanthiques, comme l'association de caféine et de silanol (dérivé méthylsilanetriol de caféine), et par exemple le produit commercialisé par Exsymol sous la dénomination caféisilane C ;
      - les composés d'origine naturelle contenant des bases xanthiques et notamment de la caféine, tels que les extraits de thé, de café, de guarana, de maté, de cola *(Cola Nitida)* et notamment l'extrait sec de fruit de guarana *(Paulina sorbilis)* contenant 8 à 10 % de caféine ;
      - l'éphédrine et ses dérivés qui peuvent notamment se retrouver à l'état naturel dans les plantes telles que le Ma Huang (Ephedra plant)
   2) les extraits végétaux et les extraits d'origine marine, qui sont soit actifs sur les récepteurs à inhiber, tels que les β-2-bloqueurs, les NPY-bloqueurs (décrits dans le brevet EP 838217), soit inhibent la synthèse des récepteurs aux LDL ou VLDL, soit actifs pour stimuler les récepteurs β et les protéines G, conduisant à l'activation de l'adénylcyclase. Comme extraits végétaux de ce type, on peut citer par exemple :
      - le *Garcinia Cambogia,*
      - les extraits de *Bupleurum chinensis,*
      - les extraits de lierre grimpant *(Hedera Helix),* d'arnica (*Arnica Montana L),* de romarin (*Rosmarinus officinalis L),* de souci *(Calendula officinalis*), de sauge *(Salvia officinalis L),* de ginseng *(Panax ginseng*), de millepertuis *(Hyperycum Perforatum),* de fragon *(Ruscus aculeatus L),* d'ulmaire *(Filipendula ulmaria L),* d'orthosiphon *(Orthosiphon Stamincus Benth*), de bouleau (*Betula alba),* de cécropia et d'arganier.
      - les extraits de ginkgo biloba,
      - les extraits de prêle,
      - les extraits d'escine,
      - les extraits de cangzhu,
      - les extraits de *chrysanthellum indicum,*
      - les extraits de dioscorés riches en diosgénine ou la diosgénine ou hécogénine pure et leurs dérivés.
      - les extraits des plantes du genre *Armeniacea, Atractylodis Platicodon, Sinom-menum, Pharbitidis, Flemingia,*
      - les extraits de *Coleus* tels que *C. Forskohlii, C. blumei, C. esquirolii, C. scutellaroïdes , C. xanthantus* et *C. Barbatus,* tel que l'extrait de racine de *Coleus Barbatus* contenant 60 % de forskoline,
      - les extraits de Ballote,
      - les extraits de *Guioa,* de *Davallia*, de *Terminalia,* de *Barringtonia,* de *Trema,* d'*Antirobia*.
         Comme extrait d'origine marine on peut citer les extraits d'algues ou de phytopancton, tels que le rhodystérol ou l'extrait de *Laminaria Digitata* commercialisé sous la dénomination PHYCOX75 par Secma, l'algue skeletonema décrite dans le brevet FR 2 782 921 ou les diatomées décrites dans le brevet FR 2774292.
   3) les peptides ou protéines
      - les peptides dérivés de l'hormone parathyroidienne tels que décrits dans les brevets FR 2 788058 et FR 2781231 de Séderma ou les peptides décrits dans le document FR 2 786 693 voire tout autre peptide ayant des propriétés lipolytiques,
      - les protamines et leurs dérivés tels que ceux décrits dans le document FR-A-2,758,724. La quantité d'actif(s) lipolytique(s) peut varier dans une large mesure et dépend de la nature de ou des actifs utilisés. De manière générale, le ou les actifs amincissants sont présents en une concentration allant de 0,001 à 20 % et de préférence de 0,1 à 10 % en poids par rapport au poids total de la composition.
- les agents agissant sur la microcirculation :
   Les actifs agissant sur la microcirculation (vasoprotecteur ou vasodilatateur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.
   La quantité de ces actifs peut varier dans une large mesure. De manière générale, ces actifs sont présents en une concentration allant de 0,01 à 15 % et de préférence de 0,05 à 10 % en poids par rapport au poids total de la composition.
- et les agents agissant sur le métabolisme énergétique des cellules ;
   Les actifs concernés sont ceux qui agissent sur le métabolisme énergétique cutané tel que, par exemple, et de façon non limitative, la synthèse d'ATP, ceux qui sont interviennent sur la chaîne respiratoire de la cellule ou sur les réserves énergétiques. On peut citer le Coenzyme Q10 (ubiquinone), le cytochrome C, la créatine ou encore la phosphocréatine.

Des composés topiques dont l'utilisation peut, dans des circonstances particulières telles qu'une peau réactive, une peau atteinte de rosacée, des concentrations élevées desdits composés..., conduire à l'apparition de rougeurs cutanées sont utilisés dans des compositions cosmétiques ou dermatologiques, bien entendu pour d'autres effets.
Ainsi, on utilise des compositions cosmétiques contenant des actifs kératolytiques, pour lutter contre le vieillissement, et notamment des actifs exfoliants ou des actifs favorisant le renouvellement cellulaire, tels que les α-hydroxy-acides (notamment acides lactique, glycolique, citrique), les β-hydroxy-acides (notamment acides salicylique, n-octanoyl-5-salicylique) et les rétinoïdes (notamment acide rétinoïque tout trans ou 13-cis, rétinol). Malheureusement, si ces actifs sont utilisés en des quantités trop importantes, ils peuvent provoquer des rougeurs cutanées et leur utilisation peut donc devoir être limitée. Il peut également s'agir de conservateurs, de tensioactifs, de parfums, de solvants ou de propulseurs.

La présence d'un antagoniste de substance P sous la forme de l'association de niacinamide et du dipeptide tyrosine-arginine dans une composition comprenant un produit susceptible de présenter un effet irritant permet d'atténuer fortement, voire de supprimer cet effet irritant.
Cela permet en outre d'augmenter la quantité de principe actif susceptible de présenter un effet irritant par rapport à la quantité de principe actif normalement utilisée, en vue d'une efficacité améliorée.

L'emploi d'antagoniste de substance P permet notamment de multiplier de 2 à 10 fois la quantité de principe actif à effet irritant par rapport à l'état de la technique, en atténuant tout ou partie des inconforts mentionnés ci-dessus.

Que leur application relève du domaine cosmétique ou thérapeutique, les compositions selon l'invention peuvent être administrées par voie orale, entérale ou encore par voie topique, on préférera l'administration par voie topique.

Dans le cas d'une administration par voie orale, les compositions selon l'invention peuvent se présenter sous toute forme adaptée telle qu'une solution buvable, des gélules, dragée, capsule molle ou dure, comprimés à avaler ou à croquer, granulés à dissoudre, sirop, aliment solide ou liquide...

Par milieu physiologiquement acceptable selon l'invention, on entend un milieu compatible avec la peau, les muqueuses et/ou le cuir chevelu et ses phanères.

La composition selon l'invention peut se présenter sous les formes galéniques classiquement utilisées pour une application topique et notamment sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de nanoémulsions, de dispersions vésiculaires de type ionique et/ou non ionique, ou des dispersions cire/phase aqueuse. Ces compositions sont préparées selon les méthodes usuelles.
Elle peut également se présenter sous la forme d'un système transdermique permettant une libération active ou passive du(des) actif(s) par transdermie, par exemple de type patch ou gel patch (hydrogel).

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. Les compositions sous forme d'émulsion peuvent être exemptes d'émulsionnant, dans le cas où la composition comprend émulsionnant et/ou co-émulsionnant, ces derniers sont généralement présents dans la composition, en une proportion allant de 0,3 à 30 % en poids, et de préférence de 0,5 à 20 % en poids par rapport au poids total de la composition.

La composition de l'invention peut ainsi constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un composé parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps. Elle peut, en outre, se présenter sous forme de shampooing traitant ou non, colorant ou non, et d'après-shampooing.

La composition selon l'invention peut également constituer une composition cosmétique colorée et notamment une composition de maquillage de la peau, des fibres kératiniques (cheveux ou cils) et/ou des muqueuses, en particulier un fond de teint, un blush, un fard à joues ou à paupières, un composé anti-cernes en stick, un rouge à lèvres ou un brillant à lèvres, présentant éventuellement des propriétés de soin ou de traitement. De préférence, il pourra s'agir d'une composition de maquillage colorée (beige ou verte) destinée à corriger la couleur du teint.

L'invention sera illustrée par les exemples non limitatifs suivants.

### Exemple 1 : Évaluation histologique de la surface moyenne des capillaires sanguins

Les essais des exemples 1 et 2 ci-après ont été réalisés avec la niacinamide à une concentration finale de 0,5% et le N-acetyl-Tyrosine-Arginine-decahexyl ester fourni par la société Sederma à une concentration finale de 0,1%, sa structure chimique est la suivante :

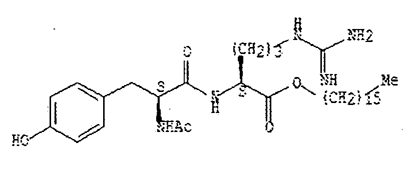

L'activité de chacun des actifs et de leur association est évaluée dans un modèle de peau humaine maintenue en survie.

Après plusieurs heures de rééquilibrage de la peau avec du milieu de culture (antibiotique + SVF), le milieu de culture est renouvelé et la substance P à 10 µM, avec ou sans chacun des actifs ou leur association, sont ajoutés au milieu de culture.
Qu'ils soient seuls ou en association, les actifs sont utilisés aux concentrations finales de 0,1% pour la niacinamide et de 0,5% pour le dipeptide.

L'analyse morphométrique de la surface (µm²) occupée par la lumière des vaisseaux est réalisée par observation microscopique sur des coupes : les fragments de peaux sont fixés dans le liquide de Bouin et inclus en paraffine puis colorés par l'hémalun-éosine. La dilatation vasculaire est évaluée par un comptage du nombre de vaisseau dilaté sur l'ensemble de la coupe histologique (16 champs au grossissement 40).
Cette analyse permet de déterminer la surface moyenne (µm²) occupée par les vaisseaux dans le derme.

Le traitement avec la substance P à 10 µM conduit à une augmentation de la surface de 63% par rapport à la peau saine (161,6+/-56,6 µm² contre 99+/-57,6 µm² pour la peau saine).

| **Traitement** | **Pourcentage de capillaires sanguins dermiques dilatés** |
|---|---|
| Peau témoin | 99+/-57.5 |
| Peau + substance P | 161,6+/-56.6 |
| Peau + substance P + niacinamide | 108+/-27.6 |
| Peau + substance P + dipeptide | 103,16+/-34 |
| Peau + substance P + niacinamide + dipeptide | 69,1+/-14.8 |

La surface des capillaires dilatés est significativement diminuée par rapport aux peaux traitées par la substance P, après application de chacun des deux actifs (pour chacun p<0,05).
L'association des deux actifs induit une diminution significative de la surface moyenne des capillaires par rapport aux peaux soumises à la substance P mais aussi par rapport à la peau témoin.

### Exemple 2 : Évaluation histologique de l'oedème dermique

A partir des coupes réalisées dans l'exemple 1, l'évaluation de l'oedème est réalisée à l'aide de scores semi-quantitatifs :
- score 0 : pas d'oedème
- score 1 : oedème très léger
- score 2 : oedème modéré
- score 3 : oedème important

Le score de la peau témoin est de 0,86+/-0,7. Le traitement avec la substance P conduit à une augmentation du score de l'oedème de 121% (1,9+/-0,64).

| **Traitement** | **Pourcentage de capillaires dermiques dilatés** |
|---|---|
| Peau témoin | 0,86+/-0.7 |
| Peau + substance P | 1,9+/-0.7 |
| Peau + substance P + niacinamide | 1,03+/-0,64 |
| Peau + substance P + dipeptide | 1+/-0,6 |
| Peau + substance P + niacinamide + dipeptide | 0,78+/-0.3 |

Le traitement par chacun des actifs permet de diminuer l'oedème de façon significative par rapport aux peaux stimulées par la substance P (p<0,05).
L'association des deux actifs induit également une diminution de l'oedème dermique par rapport aux peaux soumise à la substance P et par rapport à la peau témoin.

Pour chacun de ces deux exemples, l'application de l'un ou l'autre des actifs niacinamide et dipeptide permettent de ramener le paramètre mesuré à un état correspondant au témoin.
L'association de niacinamide et de dipeptide permet quant à elle de diminuer significativement le diamètre de dilatation des vaisseaux sanguins et conduit également à une diminution des oedèmes cutanés et ce avec une activité supérieure par rapport à chacun des actifs pris séparément.
En conclusion, l'effet de l'association niacinamide et dipeptide est significativement amélioré par rapport à l'effet de chacun des actifs pris séparément et démontre bien les avantages découlant de l'association de ces deux actifs.

### Exemple 3 : Compositions

### Emulsion huile dans eau

| | | |
|---|---|---|
| **A** - | Eau | QSP 100 % |
| | Conservateurs | 0.5 % |
| | Glycérine | 5 % |
| | Niacinamide | 2,5 % |
| | Dipeptide | 2,5 % |
| | Caféine | 0.3 % |
| | Dipeptide | 1% |

| | | |
|---|---|---|
| **B -** | Glyceryl stearate et PEG-100 stearate | 3 % |
| | Acide stérarique | 1 % |
| | Alcool cétylique | 2 % |
| | Isononyl isononanoate | 10 % |
| | Acrylate copolymer | 0.3 % |

| | | |
|---|---|---|
| **C -** | Cyclohexasiloxane | 5 % |
| | Carbomer | 0.3 % |
| | Gomme de xanthane | 0.2 % |

### Mode opératoire

La phase A est chauffée à 85°C sous agitation jusqu'à obtention d'une phase limpide puis est ramenée à 70°C.
La phase B est chauffée à 70°C et homogénéisée sous agitation puis ajoutée à la phase A pour la mise en émulsion sous agitation. Le tout est ramené à 30°C.
La gomme de xanthane et le carbomer sont dispersés dans le cyclohexacyloxane à température ambiante jusqu'à obtention d'une phase homogène puis ajoutée au mélange A+B.
Le tout est ramené à température ambiante.
On obtient une émulsion homogène destinée traiter les problèmes de rougeur cutanée.

### Lotion capillaire antichute

- dextran sulfate 500kD 0,5%
- Aminexil 1,5%
- Acide salicylique 0,2%
- dipeptide 2%
- niacinamide 3%
- Propylène glycol 30%
- alcool éthylique 40,5%
- eau qsp 100%

### Composition de peeling

- dextran sulfate 1000kD 1 %
- Acide glycolique 10%
- Acide lactique 10%
- dipeptide 3%
- niacinamide 2%
- Calcium-D-pantéthéine sulfonate 1%
- Eau 15%
- Ethanol qsp 100%

### Patch dépigmentant

On prépare un gel de composition suivante :
- vitamine C 2 %
- acide salycilique 0,2 %
- niacinamide 4 %
- dipeptide 4 %
- Méthyl sulfonyl méthane 5 %
- Glycérine 5 %
- Alginate de sodium 10 %
- Alcool polyvinylique 10 %
- Polyacrylate de sodium 5 % (Aronvis S de NIHON JUNYAKU)
- Conservateurs 1 %
- Eau qsp 100%

Après mélange des composants, le gel ainsi obtenu est enduit sur un support en non-tissé puis découpé pour former un patch.

## Revendications

1. Utilisation de l'association de niacinamide et du dipeptide tyrosine-arginine correspondant à la formule générale suivante : R1-L-Tyr-L-Arg-R2 avec
- R1 est un atome d'hydrogène ou un groupe -R3-C=O et R3 est une chaîne alkyle de 1 à 20 atomes de carbones, linéaire ou ramifiée, saturée ou insaturée, éventuellement hydroxylée, ou un groupe aryle, un groupe aryle-alkyle, un groupe alkyloxy ou un groupe arylalkyloxy et
- R2 est une fonction hydroxyle ; un groupe -O-R4 avec R4 est une chaîne alkyle de 1 à 20 atomes de carbones ; un groupe -NH2 ou -NHX avec X est une chaîne alkyle de 1 à 4 atomes de carbones, pour prévenir et/ou diminuer les rougeurs et/ou gonflements cutanés.

2. Utilisation selon la revendication 1, pour prévenir et/ou atténuer l'intensité des rougeurs cutanées, éclaircir et/ou uniformiser le teint et/ou masquer les rougeurs de surface et/ou estomper la microcirculation apparente.

3. Utilisation selon la revendication 1, pour éviter un aspect gonflé de la peau et/ou affiner la silhouette, le cou et/ou l'ovale du visage et/ou diminuer les poches sous les yeux et/ou traiter les chevilles et les jambes gonflées.

4. Utilisation de l'association de niacinamide et du dipeptide tyrosine-arginine correspondant à la formule générale suivante : R1-L-Tyr-L-Arg-R2 avec
- R1 est un atome d'hydrogène ou un groupe -R3-C=O et R3 est une chaîne alkyle de 1 à 20 atomes de carbones, linéaire ou ramifiée, saturée ou insaturée, éventuellement hydroxylée, ou un groupe aryle, un groupe aryle-alkyle, un groupe alkyloxy ou un groupe arylalkyloxy et
- R2 est une fonction hydroxyle ; un groupe -O-R4 avec R4 est une chaîne alkyle de 1 à 20 atomes de carbones ; un groupe -NH2 ou -NHX avec X est une chaîne alkyle de 1 à 4 atomes de carbones, pour traiter les peaux et/ou les cuirs chevelus sensibles.

5. Utilisation selon la revendication 4, pour traiter les peaux intolérantes ou irritables.

6. Utilisation selon l'une quelconque des revendications 4 ou 5, pour prévenir et/ou diminuer les sensations dysesthésiques.

7. Utilisation selon l'une quelconque des revendications 4 à 6, pour prévenir et/ou diminuer les picotements et/ou les fourmillements et/ou les démangeaisons et/ou les échauffements et/ou l'inconfort cutané et/ou les tiraillements de la peau.

8. Utilisation de niacinamide et du dipeptide tyrosine-arginine correspondant à la formule générale suivante : R1-L-Tyr-L-Arg-R2 avec
- R1 est un atome d'hydrogène ou un groupe -R3-C=O et R3 est une chaîne alkyle de 1 à 20 atomes de carbones, linéaire ou ramifiée, saturée ou insaturée, éventuellement hydroxylée, ou un groupe aryle, un groupe aryle-alkyle, un groupe alkyloxy ou un groupe arylalkyloxy et
- R2 est une fonction hydroxyle ; un groupe -O-R4 avec R4 est une chaîne alkyle de 1 à 20 atomes de carbones ; un groupe -NH2 ou -NHX avec X est une chaîne alkyle de 1 à 4 atomes de carbones, pour la préparation d'une composition destinée au traitement des désordres associés à un excès de synthèse et/ou de libération de substance P, lesdits désordres étant des désordres cutanés choisis parmi l'urticaire, les dermatites eczémateuses, la rosacée, le psoriasis, l'herpes, les photodermatoses, la dermatite atopique, la dermatite de contact, le lichen, les prurigos, les maladies prurigineuses, les fibroses, les troubles de la maturation du collagène, la sclérodermie, l'eczéma.

9. Utilisation selon la revendication 8, **caractérisée en ce que** le désordre cutané est le premier et/ou le deuxième stade de la rosacée.

10. Utilisation selon l'une quelconque des revendications précédentes, **caractérisée en ce que** ledit dipeptide est le N-acetyl-Tyrosine-Arginine-O-hexadecyl ester.

11. Utilisation selon l'une quelconque des revendications précédentes, **caractérisée en ce que** la concentration en dipeptide est comprise entre 0,001% et 20%.

12. Utilisation selon l'une quelconque des revendications précédentes, **caractérisée en ce que** la concentration en niacinamide est comprise entre 0,01% et 20%.

13. Utilisation selon l'une quelconque des revendications précédentes, **caractérisée en ce que** l'association de niacinamide et dudit dipeptide tyrosine-arginine est formulée dans une composition comprenant en outre un agent apaisant choisi parmi les extraits de rose, le bisabolol, le D-panthenol, l'allantoïne, le madecassoside, les extraits de centella asiatica, le glycyrrhizinate de potassium ou la caféine.

14. Procédé cosmétique pour prévenir et/ou estomper les rougeurs et/ou les signes de la microcirculation cutanée et/ou les gonflements cutanés et/ou les peaux sensibles et/ou les irritations cutanées, **caractérisé en ce que** l'on applique sur la peau, sur le cuir chevelu et/ou sur les muqueuses, une quantité efficace de niacinamide et de dipeptide tyrosine-arginine correspondant à la formule générale suivante : R1-L-Tyr-L-Arg-R2 avec
- R1 est un atome d'hydrogène ou un groupe -R3-C=O et R3 est une chaîne alkyle de 1 à 20 atomes de carbones, linéaire ou ramifiée, saturée ou insaturée, éventuellement hydroxylée, ou un groupe aryle, un groupe aryle-alkyle, un groupe alkyloxy ou un groupe arylalkyloxy et
- R2 est une fonction hydroxyle ; un groupe -O-R4 avec R4 est une chaîne alkyle de 1 à 20 atomes de carbones ; un groupe -NH2 ou -NHX avec X est une chaîne alkyle de 1 à 4 atomes de carbones.

## Patentansprüche

1. Verwendung der Kombination von Niacinamid und des Dipeptids Tyrosin-Arginin entsprechend der folgenden allgemeinen Formel: R1-L-Tyr-L-Arg-R2, wobei
- R1 ein Wasserstoffatom oder eine Gruppe -R3-C=O und R3 eine geradkettige oder verzweigte, gesättigte oder ungesättigte, gegebenenfalls hydroxylierte Alkylkette mit 1 bis 20 Kohlenstoffatomen oder eine Arylgruppe, eine Arylalkylgruppe, eine Alkyloxygruppe oder eine Arylalkyloxygruppe bedeutet und
- R2 eine Hydroxylfunktion; eine Gruppe -O-R4, wobei R4 eine Alkylkette mit 1 bis 20 Kohlenstoffatomen bedeutet; eine Gruppe -NH2 oder -NHX, wobei X eine Alkylkette mit 1 bis 4 Kohlenstoffatomen bedeutet, bedeutet,
zum Vorbeugen und/oder Verringern von Hautrötungen und/oder Hautschwellungen.

2. Verwendung nach Anspruch 1 zur Vorbeugung und/oder Linderung der Intensität von Hautrötungen, Aufhellen und/oder Verbessern der Ebenmäßigkeit des Teints und/oder Maskieren von Oberflächenrötungen und/oder Mildern der sichtbaren Mikrodurchblutung.

3. Verwendung nach Anspruch 1 zum Vermeiden eines angeschwollenen Aussehens der Haut und/oder Verbesserung der Konturen, des Halses und/oder des Gesichtsovals und/oder zur Verringerung der Tränensäcke unter den Augen und/oder zur Behandlung von angeschwollenen Fesseln und Beinen.

4. Verwendung der Kombination von Niacinamid und des Dipeptids Tyrosin-Arginin entsprechend der folgenden allgemeinen Formel: R1-L-Tyr-L-Arg-R2, wobei
- R1 ein Wasserstoffatom oder eine Gruppe -R3-C=O und R3 eine geradkettige oder verzweigte, gesättigte oder ungesättigte, gegebenenfalls hydroxylierte Alkylkette mit 1 bis 20 Kohlenstoffatomen oder eine Arylgruppe, eine Arylalkylgruppe, eine Alkyloxygruppe oder eine Arylalkyloxygruppe bedeutet und
- R2 eine Hydroxylfunktion; eine Gruppe -O-R4, wobei R4 eine Alkylkette mit 1 bis 20 Kohlenstoffatomen bedeutet; eine Gruppe -NH2 oder -NHX, wobei X eine Alkylkette mit 1 bis 4 Kohlenstoffatomen bedeutet, bedeutet,
zur Behandlung von empfindlicher Haut und/oder Kopfhaut.

5. Verwendung nach Anspruch 4 zur Behandlung von unverträglicher oder reizbarer Haut.

6. Verwendung nach Anspruch 4 oder 5 zur Vorbeugung und/oder Verringerung von dysästhetischen Empfindungen.

7. Verwendung nach einem der Ansprüche 4 bis 6 zur Vorbeugung und/oder Verringerung des Prickelns und/oder des Kribbelns und/oder von Juckreiz und/oder von Wärmegefühl und/oder von mangelndem Hautkomfort und/oder des Ziehens der Haut.

8. Verwendung von Niacinamid und des Dipeptids Tyrosin-Arginin entsprechend der folgenden allgemeinen Formel: R1-L-Tyr-L-Arg-R2, wobei
- R1 ein Wasserstoffatom oder eine Gruppe -R3-C=O und R3 eine geradkettige oder verzweigte, gesättigte oder ungesättigte, gegebenenfalls hydroxylierte Alkylkette mit 1 bis 20 Kohlenstoffatomen oder eine Arylgruppe, eine Arylalkylgruppe, eine Alkyloxygruppe oder eine Arylalkyloxygruppe bedeutet und
- R2 eine Hydroxylfunktion; eine Gruppe -O-R4, wobei R4 eine Alkylkette mit 1 bis 20 Kohlenstoffatomen bedeutet; eine Gruppe -NH2 oder -NHX, wobei X eine Alkylkette mit 1 bis 4 Kohlenstoffatomen bedeutet, bedeutet,
für die Herstellung einer Zusammensetzung, die für die Behandlung von Leiden, die mit übermäßiger Synthese und/oder Freisetzung von Substanz P einhergehen, wobei es sich bei den Leiden um Hautleiden aus der Reihe Urticaria, ekzemartige Dermatitiden, Rosacea, Schuppenflechte, Herpes, lichtbedingte Dermatosen, atropische Dermatitis, Kontaktdermatitis, Lichen, Juckreiz, juckende Krankheiten, Fibrosen, Collagenreifungsprobleme, Sklerodermie und Ekzem handelt.

9. Verwendung nach Anspruch 8, **dadurch gekennzeichnet, dass** es sich bei dem Hautleiden um das erste und/oder zweite Stadium von Rosacea handelt.

10. Verwendung nach einem der vorhergehenden Ansprüche, **dadurch gekennzeichnet, dass** es sich bei dem Dipeptid um N-Acetyl-tyrosin-arginin-O-hexadecylester handelt.

11. Verwendung nach einem der vorhergehenden Ansprüche, **dadurch gekennzeichnet, dass** die Dipeptidkonzentration zwischen 0,001% und 20% beträgt.

12. Verwendung nach einem der vorhergehenden Ansprüche, **dadurch gekennzeichnet, dass** die Niacinamidkonzentration zwischen 0,01% und 20% beträgt.

13. Verwendung nach einem der vorhergehenden Ansprüche, **dadurch gekennzeichnet, dass** die Kombination von Niacinamid und dem Tyrosin-Arginin-Dipeptid in einer Zusammensetzung formuliert ist, die weiterhin einen beruhigenden Wirkstoff aus der Reihe Rosenextrakte, Bisabolol, D-Panthenol, Allantoin, Madecassosid, Centella-asiatica-Extrakte, Kalium-Glycyrrhizinat oder Koffein umfasst.

14. Kosmetisches Verfahren zur Vorbeugung und/oder Linderung von Rötungen und/oder Anzeichen der Mikrodurchblutung der Haut und/oder Hautschwellungen und/oder sensibler Haut und/oder von Hautreizungen, **dadurch gekennzeichnet, dass** man auf die Haut, die Kopfhaut und/oder die Schleimhäute eine wirksame Menge Niacinamid und Tyrosin-Arginin-dipeptid entsprechend der folgenden allgemeinen Formel R1-L-Tyr-L-Arg-R2, wobei
- R1 ein Wasserstoffatom oder eine Gruppe -R3-C=O und R3 eine geradkettige oder verzweigte, gesättigte oder ungesättigte, gegebenenfalls hydroxylierte Alkylkette mit 1 bis 20 Kohlenstoffatomen oder eine Arylgruppe, eine Arylalkylgruppe, eine Alkyloxygruppe oder eine Arylalkyloxygruppe bedeutet und
- R2 eine Hydroxylfunktion; eine Gruppe -O-R4, wobei R4 eine Alkylkette mit 1 bis 20 Kohlenstoffatomen bedeutet; eine Gruppe -NH2 oder -NHX, wobei X eine Alkylkette mit 1 bis 4 Kohlenstoffatomen bedeutet, bedeutet, aufträgt.

## Claims

1. Use of the combination of niacinamide and of the tyrosine-arginine dipeptide corresponding to the following general formula: R1-L-Tyr-Z-Arg-R2, with
- R1 a hydrogen atom or an -R3-C=O group and R3 an optionally hydroxylated, saturated or unsaturated and linear or branched alkyl chain of 1 to 20 carbon atoms, or an aryl group, an arylalkyl group, an alkyloxy group or an arylalkyloxy group, and
- R2 a hydroxyl functional group; an -0-R4 group with R4 an alkyl chain of 1 to 20 carbon atoms; or an -NH₂ or -NHX group with X an alkyl chain of 1 to 4 carbon atoms,
for preventing and/or decreasing red blotches and/or puffiness of the skin.

2. Use according to Claim 1, for preventing and/or reducing the intensity of red blotches on the skin, lightening and/or making uniform the complexion and/or masking surface red blotches and/or fading out noticeable microcirculation.

3. Use according to Claim 1, for preventing a puffy appearance of the skin and/or making the figure, the neck and/or the oval of the face more slender and/or decreasing bags under the eyes and/or treating puffy ankles and legs.

4. Use of the combination of niacinamide and of the tyrosine-arginine dipeptide corresponding to the following general formula: R1-L-Tyr-L-Arg-R2, with
- R1 a hydrogen atom or an -R3-C=O group and R3 an optionally hydroxylated, saturated or unsaturated and linear or branched alkyl chain of 1 to 20 carbon atoms, or an aryl group, an arylalkyl group, an alkyloxy group or an arylalkyloxy group, and
- R2 a hydroxyl functional group; an -O-R4 group with R4 an alkyl chain of 1 to 20 carbon atoms; or an -NH₂ or -NHX group with X an alkyl chain of 1 to 4 carbon atoms,
for treating sensitive skin and/or sensitive scalps.

5. Use according to Claim 4, for treating intolerant or irritable skin.

6. Use according to either one of Claims 4 and 5, for preventing and/or decreasing dysaesthetic sensations.

7. Use according to any one of Claims 4 to 6, for preventing and/or decreasing stinging and/or pins and needles and/or itching and/or overheating and/or skin discomfort and/or tautness of the skin.

8. Use of niacinamide and of the tyrosine-arginine dipeptide corresponding to the following general formula: R1-L-Tyr-L-Arg-R2, with
- R1 a hydrogen atom or an -R3-C=O group and R3 an optionally hydroxylated, saturated or unsaturated and linear or branched alkyl chain of 1 to 20 carbon atoms, or an aryl group, an arylalkyl group, an alkyloxy group or an arylalkyloxy group, and
- R2 a hydroxyl functional group; an -O-R4 group with R4 an alkyl chain of 1 to 20 carbon atoms; or an -NH₂ or -NHX group with X an alkyl chain of 1 to 4 carbon atoms,
for the preparation of a composition for use in the treatment of disorders associated with an excess synthesis and/or release of substance P, the said disorders being skin disorders chosen from urticaria, eczematous dermatitis, rosacea, psoriasis, herpes, photodermatoses, atopic dermatitis, contact dermatitis, lichen, prurigos, pruriginous diseases, fibroses, collagen maturation conditions, scleroderma and eczema.

9. Use according to Claim 8, **characterized in that** the skin disorder is the first and/or second stage of rosacea.

10. Use according to any one of the preceding claims, **characterized in that** the said dipeptide is N-acetyl-Tyrosine-Arginine-0-hexadecyl ester.

11. Use according to any one of the preceding claims, **characterized in that** the concentration of dipeptide is between 0.001% and 20%.

12. Use according to any one of the preceding claims, **characterized in that** the concentration of niacinamide is between 0.01% and 20%.

13. Use according to any one of the preceding claims, **characterized in that** the combination of niacinamide and of the said tyrosine-arginine dipeptide is formulated in a composition also comprising a calmative chosen from extracts of rose, bisabolol, D-panthenol, allantoin, madecassoside, extracts of *Centella asiatica*, potassium glycyrrhizinate or caffeine.

14. Cosmetic process for preventing and/or fading out red blotches and/or the signs of skin microcirculation and/or puffiness of the skin and/or sensitive skin and/or skin irritations, **characterized in that** an effective amount of niacinamide and of tyrosine-arginine dipeptide corresponding to the following general formula: R1-L-Tyr-L-Arg-R2, with
- R1 a hydrogen atom or an -R3-C=O group and R3 an optionally hydroxylated, saturated or unsaturated and linear or branched alkyl chain of 1 to 20 carbon atoms, or an aryl group, an arylalkyl group, an alkyloxy group or an arylalkyloxy group, and
- R2 a hydroxyl functional group; an -O-R4 group with R4 an alkyl chain of 1 to 20 carbon atoms; or an -NH₂ or -NHX group with X an alkyl chain of 1 to 4 carbon atoms,
is applied to the skin, to the scalp and/or to the mucous membranes.
